# EUROPEAN PATENT SPECIFICATION

(11) **EP 2 629 820 B1**
(45) Date of publication and mention of the grant of the patent: **07.08.2019**
(21) Application number: 10858736.1
(22) Date of filing: 20.10.2010
(51) Int. Cl.: A61M 11/02, A61M 11/06, A61M 25/10, B05B 1/10, A61M 11/00, A61M 16/04

(54) **MINIATURE FLUID ATOMIZER**
MINIATUR-FLUIDZERSTÄUBER
ATOMISEUR DE FLUIDE MINIATURE

(43) Date of publication of application: 28.08.2013
(73) Proprietor: Teleflex Medical Incorporated, Wayne, PA 19087 (US)
(72) Inventor: CROLL, Perry, W., Salt Lake City, UT 84092 (US); DENTON, Marshall, T., Salt Lake City, UT 84105 (US); CHRISTENSEN, Mark, A., Salt Lake City, UT 84102 (US); TRAN, Huy, N., Riverton, UT 84065 (US)
(74) Representative: Jacob, Reuben Ellis
(86) International application number: PCT/US2010/002805
(87) International publication number: WO 2012/054013

(56) References cited:
- WO-A1-89/09654
- WO-A1-2009/035646
- US-A- 5 370 318
- US-A- 5 642 730
- US-A1- 2003 172 934
- US-A1- 2005 131 357
- US-B2- 6 698 429
- US-B2- 7 472 705

## Description

### TECHNICAL FIELD

The invention relates generally to atomizing nozzles and devices that dispense treatment fluids in a misted or dispersed, small particle size, form. Certain devices constructed described herein are particularly suitable for use in devices adapted for insertion through small-bore conduits to apply misted fluid onto interior portions of a human patient.

### BACKGROUND

Details of the principles of operation and construction of certain operable atomizing nozzles are disclosed in United States Patent 6,698,429, titled "MEDICAL ATOMIZER," issued March 2, 2004, to Perry W. Croll, et al., the entire disclosure of which is hereby incorporated by reference. It is believed that the inventors' prior work in small size atomizing nozzles, disclosed in the aforementioned '429 patent, constitutes the most advanced "state of the art" in the area of disposable atomizing nozzle components having a small cross-section profile permitting their insertion into medically related tubes and orifices.

Bodies of atomizer components structured according to the disclosure of Croll et al. include an injection molded socket into which is adhesively bonded an extension tube member. Issues inherent in the injection molding process effectively limit a size (*e.g.,* diameter) that can reliably be manufactured for such socket. It is believed that a minimum socket wall thickness that can be reliably injection molded is about 0.015 inch (0.381 mm). Therefore, a realistic minimum socket diameter is about 0.030 inch (0.76 mm) larger than the outside diameter of an extension member. The maximum cross-sectional body diameter of the atomizer components disclosed in Croll et al. is even larger than the minimum outside socket diameter.

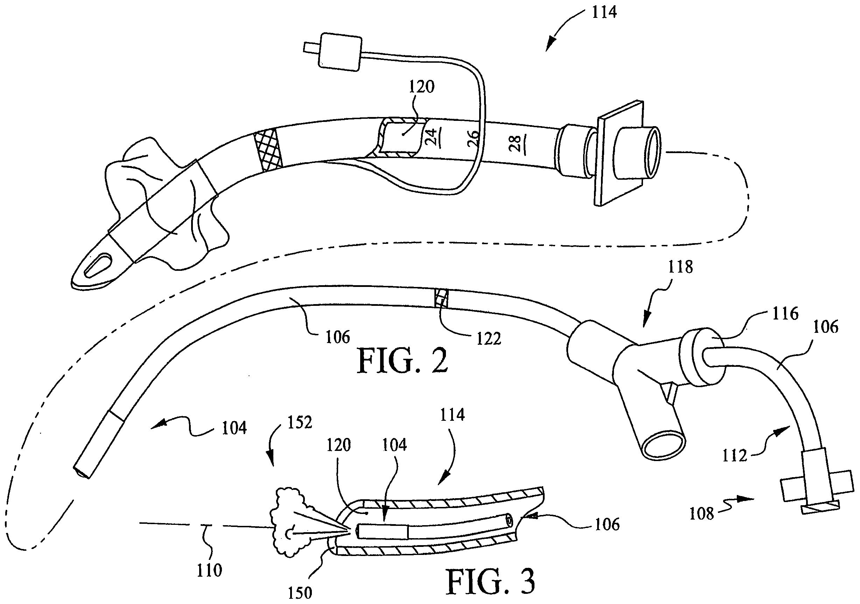

As disclosed in Croll et al. at col. 5, lines 47-56, the nominal diameter of the nozzle body D_{b} is about 0.2 inch (0.5 cm), and the diameter of the cylindrical extension conduit Dₑ is about 0.1 inch (0.25 cm). Such numbers produce a ratio of atomizer body diameter to extension member diameter (D_{b} / Dₑ) of 2.0. More precise direct measurements of a corresponding commercially available embodiment yield a maximum atomizer body diameter of 0.218 inch (0.55 cm) and extension member diameter of 0.121 inch (0.3 cm), producing a D_{b} / Dₑ ratio of 1.8. Corresponding direct measurements of a commercially available alternative embodiment such as is illustrated in FIG. 3 of the Croll et al. are 0.188 inch (0.5 cm) and 0.119 inch (0.3 cm), producing a D_{b} / Dₑ ratio of 1.6. It can be realized in theory that, as the atomizing nozzle diameter is reduced, or as the extension member becomes larger, the ratio of atomizer body diameter to extension member diameter may tend toward unity. However, due to the presence of the socket, the diameter of an atomizing nozzle component structured according to the disclosure of the'429 patent can never have a ratio of atomizer body diameter to extension member diameter equal to, or less than, 1.

A frontal area, or frontal cross-section, can be defined as that area in a fluid that must be displaced to accommodate straight-line passage of an object. As a further consequence of the injection molded socket, as the characteristic size of a frontal area cross-section (*e.g.,* diameter D_{b}, or span across a maximum size frontal area) of the body of an atomizer component is reduced toward and beyond a desired small characteristic size, the ratio of a round atomizer body diameter D_{b} to round extension member diameter Dₑ (or D_{b} / Dₑ) inevitably moves farther away from unity (in other words, D_{b} / Dₑ becomes increasingly greater than one).

For purpose of this disclosure, an atomizer component having a desired small characteristic size means that a round body diameter is less than about 0.2 inch (0.5 cm). In that limiting case, with minimum socket wall thickness and 0.2 inch (0.5 cm) body diameter, the ratio of a round atomizer body diameter to a round extension member diameter is 0.2/0.17 or 1.176. The D_{b} / Dₑ ratio inevitably increases (grows numerically larger from 1.176) as the diameters of the components are reduced while holding socket wall thickness at its minimum value for an injection molded part. If the extension member alone is made smaller in diameter (socket wall thickness is made greater than the minimum 0.015 inch (0.038 cm) while atomizer body diameter remains 0.2 inch (0.5 cm)), then the ratio of atomizer body diameter to extension member diameter is also correspondingly increased (grows numerically larger from 1.176).

Atomizing nozzle components may sometimes be characterized by the ratio of diameter of the atomizing component's body D_{b} to the diameter of the ejection orifice O (or D_{b} /O). Ejection orifices are typically sized small (*e.g.,* about 0.010 to 0.008 inch (0.0254 to 0.02032 cm) in diameter) to cause a sufficient pressure drop to promote a reasonable atomizing result in dispensed fluid, but still must be large enough to deliver a reasonable flow rate of, *e.g.,* treatment fluid and to resist clogging. It is believed that about 0.010 to 0.008 inch (0.0254 to 0.02032 cm) in diameter is a reasonable limit for the nominal size of operable ejection orifices.

It is axiomatic that, given a fixed orifice size, the smaller the diameter of the body D_{b}, the smaller the numeric value for the ratio D_{b} /O. The nominal body size of 0.2 inch (0.5 cm) and aforementioned sizes of ejection orifices of 0.008 and 0.10 yield D_{b}/O ratios of 25 and 20, respectively. Using the smallest commercially available body diameter of 0.188 in. (0.478 cm) in combination with the larger 0.010 inch (0.0254 cm) ejection orifice diameter (which is larger than the 0.008 inch (0.02032 cm) diameter orifice actually present in such device), a D_{b} /O ratio of 18.8 is calculated. It is believed that all disposable prior art atomizers have a D_{b} /O ratio greater than 18.8.

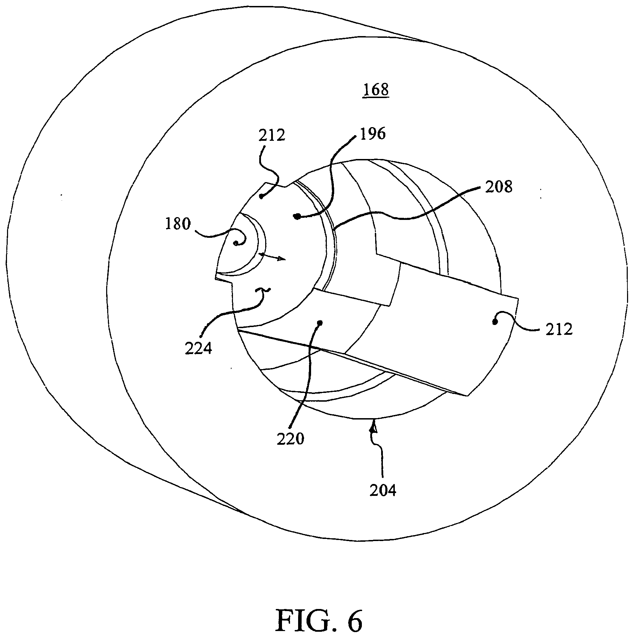

Atomizing nozzle components may sometimes be characterized by the ratio of the outside diameter (or maximum characteristic size) of an extension member to the diameter of the ejection orifice through which is expelled treatment fluid. A certain minimum fluid pressure is required to properly atomize a dispensed dose of fluid. The outside diameter of an extension conduit must be sufficiently large as to provide structural integrity to resist the pressure of treatment fluid upstream of the atomizing portion of the assembly. In accordance with Croll et al., certain extension conduits also may include one or more extra lumen in which to hold a deformable member, so the diameter of such extension members is even larger. Commercially available atomizer components corresponding to embodiments illustrated in FIG. 3 and FIG. 6 of Croll et al. have ejection orifices sized 0.008 inch (0.0203 cm) and 0.010 inch (0.025 cm), respectively. An extension member formed from nominal 1/8 inch (0.32 cm) diameter medical tubing produces ratios of the diameter of the extension member to the diameter of the ejection orifice of 15.6 for the FIG. 3 embodiment and 12.5 for the FIG. 6 embodiment, as disclosed in Croll et al. Using the previously listed direct measurements for commercially available extension member diameters produces such ratios of 14.88 and 12.11, respectively. As the characteristic size of the extension member increases for a given size ejection orifice, the ratio of the maximum characteristic size of an extension member "Dₑ" to the size of the nozzle's ejection orifice "O" (or Dₑ /O) inherently increases. It is believed that the Dₑ /O ratio of all prior art arrangements is larger than 12.

For atomizing components (and/or extension members) that are not round, similar ratios such as: atomizer body diameter to extension member diameter, or diameter of the extension member to the diameter of the ejection orifice, or diameter of the body to diameter of the ejection orifice, may be obtained using their maximum characteristic sizes. Therefore, for purpose of this disclosure, maximum characteristic size and diameter may sometimes be used interchangeably. For convenience, the term "maximum" may sometimes even be left out, with the understanding that a proper meaning may be logically deduced in context.

Atomizing nozzle components structured according to Croll et al. inherently include a shoulder disposed at the proximal end of the atomizing component and extension member interface area. A minimum effective shoulder of about 0.015 inch (0.038 cm) in elevation is caused by the minimal wall thickness required to reliably injection mold the socket in which to receive the extension member. In practice, even larger shoulders are present in commercially available embodiments. The step change in elevation (from the outside diameter of the extension member) at the atomizer component's shoulder forms a scraping edge that can undesirably damage tissue of a patient when the atomizing nozzle component is being withdrawn.

An atomizing nozzle may undesirably snag on structure during withdrawal of the nozzle subsequent to dispensing a treatment dose inside a patient. For example, the step change socket shoulder may form a structural interference at a distal opening of a medical tube, thereby resisting re-entrance of the atomizing nozzle component into the tube. Application of additional force on the proximal end of an extension member to overcome a shoulder induced interference while withdrawing an atomizing nozzle component introduces increased risk of decoupling the atomizing nozzle component from its extension member. It would be detrimental to decouple, and leave behind, an atomizing nozzle component in the body of a patient.

The socket is essentially required to provide sufficient bonding area to resist separation of the atomizing component from the extension member under the pressure required for atomizing operation. That is, a simple butt joint at the interface between the atomizing nozzle component and extension member is believed to provide too little surface area to form a reliable connection, at least in the small size desired (generally about 0.2 inch (0.5 cm), or less, in maximum cross-sectional diameter). Further, introduction of adhesive at a butt connection surface would undesirably introduce risk of occlusion by adhesive of the fluid delivery conduit. For the reasons listed in this and the preceding paragraphs, the ratio of the outside diameter (or maximum characteristic size) of an extension member to the diameter of the ejection orifice in prior atomizers is believed to be greater than about 12.

Certain disposable atomizing nozzle components are known, although such components have a maximum body diameter that is undesirably large, *e.g.,* greater than 0.2 inch (0.5 cm). By "disposable," it is intended to mean that an atomizing nozzle component of an assembly is sufficiently low in cost as to be disposed of after a single use. Of course, a "single use" might well include causing a plurality of discharges of sub-portions of treatment fluid. However, a "disposable" atomizer component is not generally re-sterilized, and repackaged, to permit its potential resale and/or reuse on a different patient. Disposable atomizing nozzle components are typically manufactured using low-cost mass production manufacturing techniques, such as injection molding from plastic or plastic-like materials. In contrast, a non-disposable atomizing nozzle may be machined from metal on a one-off basis, or in small lot production. The as manufactured cost of a disposable atomizing nozzle component is most preferably less than about $1.00, desirably less than about $10.00, and certainly less than about $100.00, including labor and constituent materials and measured in year 2009 United States currency.

### DISCLOSURE

Provided is an atomizing nozzle having a small frontal area permitting insertion of the nozzle into small diameter medical conduits. A body of one preferred atomizing nozzle body component has a characteristic size (*.e.g.,* cross-sectional diameter) of less than about 0.2 inch (0.5 cm). The nozzle body is typically carried on an extension member, which can be transversely flexible to permit passage of a nozzle body through a tube and along a nonlinear path. Certain extension members may optionally be plastically deformable to permit orienting a discharge direction for atomized treatment fluid. Extension members may have any desired length. The nozzle body is typically connected to an extension member by way of a lap joint associated with the outside surface of the nozzle body. Preferred embodiments have a Dₑ /O ratio of less than 12.0, a D_{b} /O ratio of less than about 18, and a D_{b} / Dₑ ratio of less than about 1.1. Certain embodiments include a swirling chamber disposed immediately upstream of the ejection orifice and having a proximal chamber wall with a fluid-wetted portion configured to at least approximate a portion of a dome, or other curved surface.

Accordingly, described herein is an improved disposable atomizing nozzle component able to be advanced through a medically related tube having an inside diameter heretofore too small to accommodate the smallest known prior art disposable atomizing nozzles. Also described is an atomizing nozzle-to-extension member interface shoulder transition having less than about 0.015 inch (0.038 cm) in elevation change. Further described is a disposable atomizing nozzle component and extension assembly that may be advanced into, and retracted from, a subject while reducing the likelihood of imparting trauma to that subject. The risk of decoupling an atomizing element from an extension member as the atomizer is withdrawn from a patient is further reduced. Further provided is a disposable atomizing nozzle component that provides increased fluid dynamic efficiency.

### BRIEF DESCRIPTION OF THE DRAWINGS

In the drawings, which illustrate what are currently regarded as the best modes for carrying out the invention:
FIG. 1 is a plan view of a fluid dispensing assembly constructed according to certain principles of the invention;
FIG. 2 is a plan view of an assembly including a portion of a fluid dispensing assembly, similar to that illustrated in FIG. 1, in combination with a thoracic medical tube apparatus;
FIG. 3 is a cross-section through a distal portion of the assembly illustrated in FIG. 2, with the distal end of the fluid dispensing assembly advanced into proximity with the distal end of the medical tube apparatus;
FIG. 4 is an exploded assembly view of an embodiment structured according to certain principles of the invention;
FIG. 5 is a cross-section view of a portion of the embodiment of FIG. 4 in assembled condition;
FIG. 5A is a close-up view of a portion of structure illustrated in FIG. 5;
FIG. 6 is a view in perspective from the proximal end of an exemplary atomizer body structured according to certain principles of the invention;
FIG. 7 is a rear view of the embodiment of FIG. 6;
FIG. 8 is a cross-section view taken through section 8-8 in FIG. 7 and looking in the direction of the arrows;

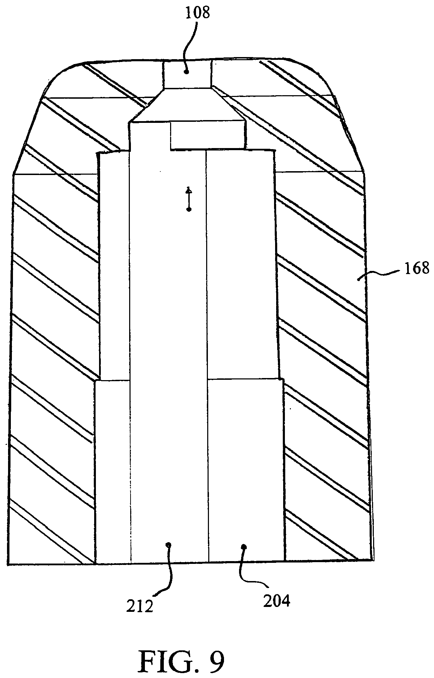

FIG. 9 is a cross-section view taken through section 9-9 in FIG. 7 and looking in the direction of the arrows;
FIG. 10 is a cross-section view in perspective of an alternative embodiment structured according to certain principles of the invention; and
FIG. 11 is a cross-section close-up view of a portion of the embodiment of FIG. 10.

### MODE(S) FOR CARRYING OUT THE INVENTION

Described is an apparatus and method for applying treatment fluid, such as anesthetic, to facilitate certain medical procedures. Non-exclusive and exemplary uses of devices structured according to certain principles of the invention include: endotracheal intubation of an awake patient; pulmonary therapy; intranasal drug delivery; sinus cavity drug delivery for delivering antibiotics; laryngo-tracheal delivery for anesthetizing the vocal cords prior to intubation; chromoendoscopy and other delivery of drugs thru an endoscope accessory port targeting the gastrointestinal mucosa; integration into an airway for intubation; delivery of surfactant at distal end of endotracheal tube to deliver drug into the lung of a neonatal patient; delivery of antibiotics or other drugs to lungs of ventilated patients via endotracheal tube; delivery of thrombin or other hemostasis agents for surgical bleeding in open or laparoscopic surgery; and delivery of, for example, bupivacaine or other fluid for pain control in open and laparoscopic surgery.

Currently preferred fluid dispensing devices are adapted to atomize expelled treatment fluid. By "atomize expelled fluid," is meant that the discharged fluid is dispersed substantially as a mist or cloud composed of very small droplets. Design variables incorporated in an atomizing nozzle include characteristic size of the discharge orifice, amount of pressure applied to the fluid upstream of the discharge orifice, and any spin chamber structural arrangement to induce fluid spin. Effective atomization requires an expelled fluid to pass through a pressure drop at a discharge orifice. Further, the expelled fluid has a rotational component of motion, (spin) about the discharge axis. Radial spread of the ejected cloud increases in correspondence with increases in the spin rate.

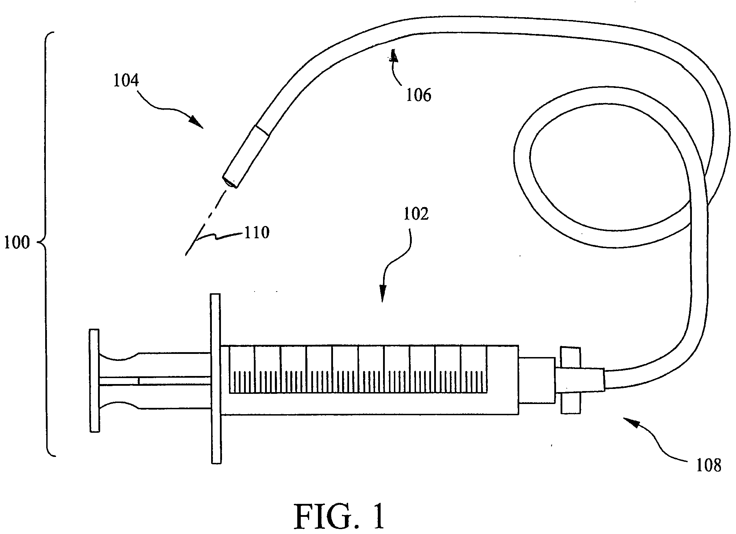

A first currently preferred dispenser for a treatment fluid is illustrated generally at 100 in FIG. 1, and includes a fluid motive source, generally 102, in combination with a dispensing nozzle, generally 104. The illustrated fluid motive source 102 in FIG. 1 is a syringe, although other arrangements effective to cause pressure on a fluid are workable. It is within contemplation alternatively to supply fluid from a pressurized or pre-pressurized canister, or even from a utility, such as a water faucet or hose bib. The illustrated dispensing nozzle 104 is a fluid atomizing nozzle operable to eject the treatment fluid as a mist or cloud. Such atomizing nozzles apply spin (about an ejection axis) to a fluid just prior to ejecting the fluid through a small diameter orifice. The discharged spinning fluid experiences a pressure drop across the exit orifice, and is thereby effectively atomized.

As illustrated in FIG. 1, the dispensing nozzle 104 may be spaced apart from the fluid motive source 102 by an extension member, generally 106. A connector, generally 108, is desirably provided to couple the nozzle 104 in communication with treatment fluid provided by the fluid motive source. The illustrated connector 108 includes a removable luer-type female coupling adapted to engage with a cooperating structure of the syringe 102. A proximal end of extension conduit 106 may be affixed to the connector 108 using known techniques, such as, e.g., adhesive bonding or welding.

A workable extension member 106 may be formed from medical grade tubing, such as the illustrated approximately 0.060 inch (0.152 cm) diameter clear plastic tubing. Such extension conduit 106 is typically transversely flexible, and may therefore be formed into the illustrated coiled shape. Certain extension conduit 106 may be structured to permit its insertion along the lumen of a medical tube, or into conduit structure of a human or animal body. In such cases, transverse flexibility of the extension conduit 106 may facilitate insertion by accommodating to a nonlinear lumen or conduit path. However, flexibility of an extension conduit 106 is not always required. In certain cases, an extension member may be substantially rigid. Sometimes, and as further detailed below, the extension member may include a plastically malleable portion that is structured to help the conduit maintain a deformed shape. In the latter case, the malleable portion is typically adapted to maintain a desired shape in the extension conduit effective to orient the spray axis 110 of the dispensing nozzle.

FIG. 2 illustrates an elongate atomized fluid delivery system, generally indicated at 112, structured according to certain principles of the invention. Certain such systems 112 are adapted to cooperate with medical tubing, such as the illustrated endotracheal tube 114. Fluid delivery system 112 includes an extension member 106 stretching between a connector 108 and a dispensing nozzle 104. Extension member 106 is disposed for slidingly sealed penetration through cap 116 of the branched adapter, generally indicated at 118, to permit advancing and retracting dispensing nozzle 104 through lumen 120. As illustrated, extension member 106 may carry indicia structure 122 to indicate a depth of insertion of the dispensing nozzle 104 into the endotracheal tube 114 and, consequently, into the patient.

FIG. 3 illustrates the dispensing nozzle 104 being advanced into proximity with the distal end 150 of endotracheal tube 114. As illustrated, it is preferred for the atomizing nozzle 104 to dispense treatment fluid as a cloud, generally 152, having a transverse diameter greater than the diameter of the tube 114. Therefore, a topical anesthetic may be applied to assist during intubation of the endotracheal tube 114.

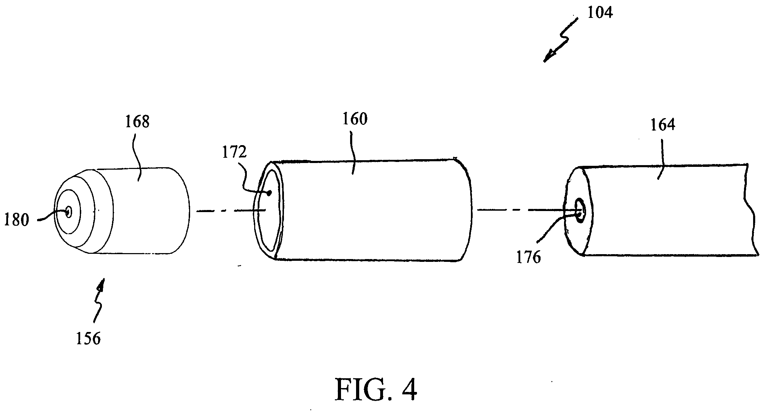

One currently preferred arrangement forming a dispensing nozzle 104 is illustrated in FIG. 4. The atomizer component, generally 156, is connected to extension conduit 160 by way of coupling 164. A workable coupling may be formed from relatively thin-walled tubing. One workable tubing includes extruded polyimide tubing having a nominal outside diameter of about 0.069 inch (0.18 cm), and a nominal inside diameter of about 0.0615 inch (0.156 cm). Such tubing may be commercially obtained from IWG High Performance Conductors, Inc. of Inman, SC, US.

The outside diameter of illustrated atomizer body 168 (FIG. 4) is sized to form a slip fit inside lumen 172 of coupling 160. Similarly, the outside diameter of extension member 164 is sized in harmony with lumen 172 to form a slip fit. Because both the body 168 and extension member 106 have the same diameter, the D_{b}/Dₑ ratio is unity (1.0). On assembly, coupling 160 forms a lap joint with each of the body 168 and a distal portion of extension member 164. The components are typically bonded together with an adhesive, such as an UV-cured adhesive or other fixant operable to form a pressure- and fluid-resistant connection. Treatment fluid delivered through fluid delivery conduit 176 is therefore confined to flow distally through ejection orifice 180.

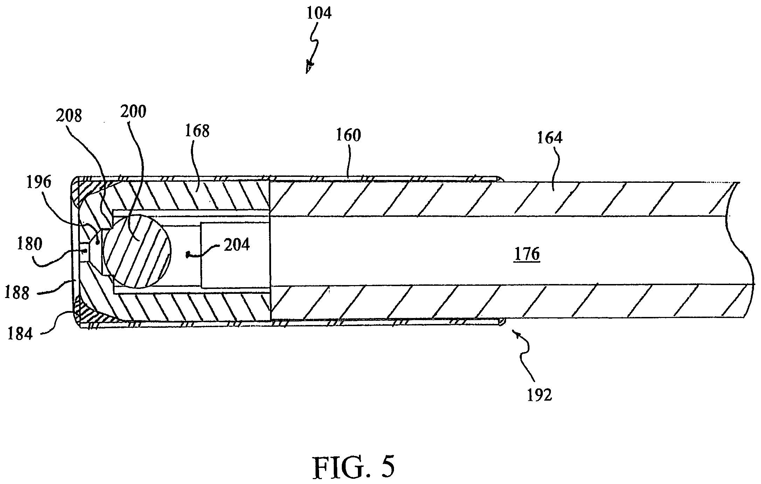

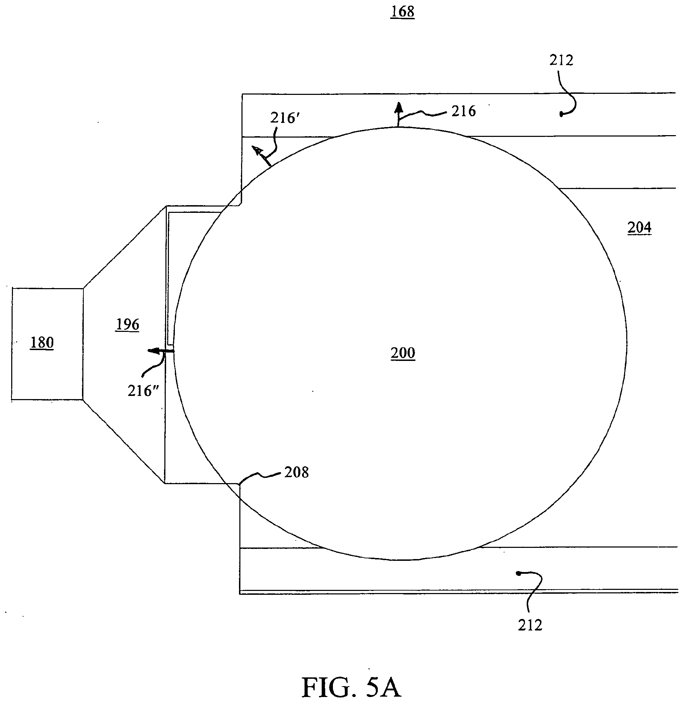

FIG. 5 illustrates the components of FIG. 4 in assembled position. Desirably, an annular plug 184 is formed by the adhesive 188. The annular plug 184 helps to resist separation of atomizer body 168 from an installed position in coupling 164 while dispensing a dose of treatment fluid. It is further desirable for the adhesive 188, or structure of a distal end of atomizer body 168, to form a blunted tip for the distal end of dispensing nozzle 104.

It is also desirable for adhesive 188 to form a transition ramp, generally indicated at 192, at the proximal end of coupling 164. Such a transition ramp resists snagging (of the small shoulder formed by the thickness of the coupling 164), during withdrawal of the nozzle 104. A smooth transition ramp 192 desirably resists scraping tissue from a patient's conduit structure. A smooth transition ramp 192 also desirably avoids a structural interference with the shoulder of a distal opening of a medical conduit through which the atomizer 104 may be retracted.

With reference now to FIGs. 5 and 5A, a swirling chamber 196 (sometimes called a "turbine chamber") is disposed immediately upstream of fluid discharge orifice 180. A proximal surface of swirling chamber 196 is formed by wall element 200, which is a ball in the illustrated embodiment. In currently preferred embodiments, ball 200 is press fit into receiving bore 204. It is currently preferred to form a press fit engagement between a portion of the ball 200 and the shoulder 208 formed in body 168. Therefore, the structure illustrated in FIG. 5A is shown to overlap by a tiny amount at corner 208. It is to be understood that such is merely to illustrate a preferred assembly arrangement. A slip fit of a wall element 200, such as a ball, in receiving bore 204 is also workable, because fluid flow inherently drives the wall element toward the swirling chamber 196. It is desirable to arrange the wall element 200 such that fluid enters the turbine chamber 196 in a way that promotes fluid spin prior to fluid ejection.

With reference to FIG. 5A, a portion of the proximal wall of swirling chamber 196 extends along a distal portion of one or more fluid channel 212. In the illustrated embodiment, two fluid channels 212 are provided. However, it is within contemplation to provide a single channel 212, or alternatively, a further plurality of channels. Desirably, a vector normal to the fluid contacting surface of a wall element 200 gradually changes from pointing substantially in a transverse direction to pointing substantially in a distal direction as the vector progresses from a proximal position of the swirling chamber 196 (indicated at vector 216), past an intermediate position (indicated at vector 216'), and toward a distal position (indicated at vector 216") along the guide surface. It is believed that such transition improves fluid dynamic properties of the discharge nozzle formed by body 168 and a wall element, such as wall element 200.

As illustrated, a wall element 200 may be formed from a spherical element, such as a ball. It is within contemplation that a wall element 200 could be a curved portion carried on a distal end of a cylinder, or by some other carrier having a different shape. The currently preferred wall element 200 is formed by a series 316 stainless steel ball having an outside diameter of 1/32 inch (0.76 mm), commercially available from worldwideweb.precisionballs.com. Of course, other elements of composition are also workable.

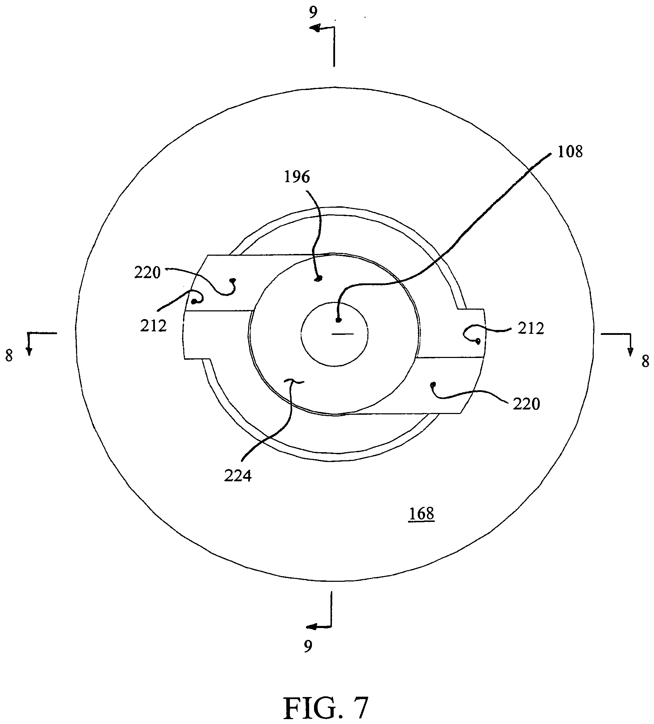

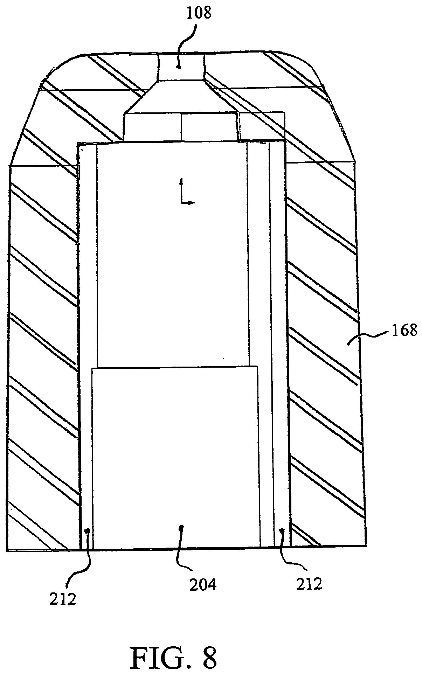

Additional details of a workable atomizer body 168 will now be discussed with reference to FIGs. 6-8. As perhaps best illustrated in FIGs. 6 and 7, treatment fluid flows distally along a fluid channel 212, then enters swirling chamber 196 by way of turbine port 220. Fluid in the swirling chamber is trapped between a wall element (not illustrated) and surface 224 of the exit cone. Therefore, fluid spirals through the chamber 196 and exits through ejection orifice 180.

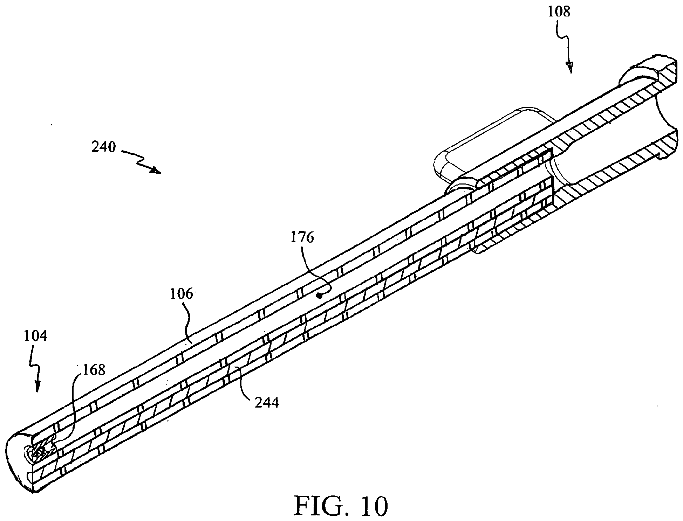

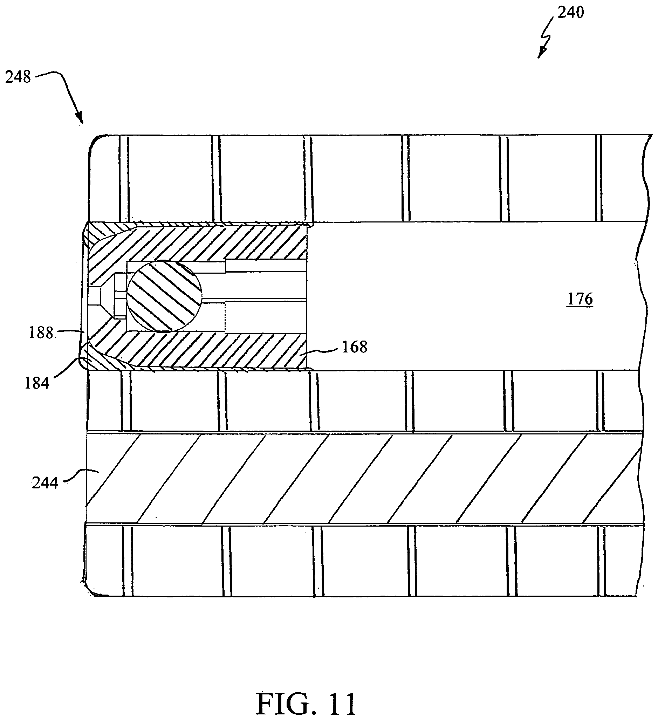

A second atomizer assembly structured according to certain principles of the invention is indicated generally at 240 in FIGs. 10 and 11. Assembly 240 includes an atomizing nozzle 104 that is spaced apart from a connector 108 by way of extension member 106'. The outside diameter of a representative extension member 106' is about 1/8 inch (0.3 cm). In contrast to the embodiment 100 (*e.g.,* see FIGs. 4 and 5), atomizer body 168 of assembly 240 is installed using a lap joint formed directly between an outside surface of body 168 and an inside surface of fluid delivery conduit 176.

Of note, embodiments of the type illustrated in FIGs. 10 and 11 inherently have a D_{b}/Dₑ ratio of less than unity (1.0). Such is believed to distinguish such embodiments over all previously developed atomizing nozzles. In the combination of an atomizing body 168 having an outside diameter of 0.060, and a nominally 1/8 inch (0.3 cm) outside diameter extension member, a D_{b}/Dₑ ratio of 0.48 may be calculated. In such an atomizing body having an ejection orifice having a diameter of 0.008 inch (0.02 cm), the D_{b}/O ratio is calculated to be 7.5. If its ejection orifice were increased to 0.010 inch (0.254 cm), the ratio would be even less: 6.0. In contrast, as previously set forth, it is believed that all previous atomizing nozzles have a D_{b}/O ratio larger than 18.8.

The outside diameter of atomizer body 168 may be of any desired size that may be manufactured. It is preferred to injection mold atomizer bodies 168 from medical grade plastic, such as, for example, polycarbonate. Of course, the inside diameter of fluid delivery conduit 176 is typically sized in agreement with the size of body 168 to permit their assembly. It is currently preferred for body 168 to form a slight press-fit inside fluid delivery conduit 176, to facilitate assembly. Similar to assembly 100, an annular ring 184 of adhesive 188 may be provided to resist decoupling the body 168 from the lumen 176. Note that the body 168 and conduit 176 are not required to be round, although such construction is more simple.

The extension member of certain assemblies may optionally include a plastically malleable portion that is structured to help the fluid delivery conduit 176 maintain a deformed shape, and thereby orient a discharge axis 110 (*e.g.,* see axis 110 in FIG. 3). As illustrated in FIGs. 10 and 11, a malleable metal wire 244 is disposed in a second lumen inside extension member 106'. Typically, at least one end of the wire 244 is affixed to the extension member to prevent its migration out of an assembled position. Adhesive attachment is currently preferred.

Wire 244 may be deformed as desired to orient a discharge direction of atomized treatment fluid. It is within contemplation that a deformable element may be associated with an extension member in alternative ways, such as by providing a plastically deformable extension element, adhering a deformable element to an exterior surface of a flexible extension member, spiraling a deformable element around the external surface of the extension member, locating the malleable element inside the fluid delivery conduit 176, or using alternative arrangements well within the capability of one of ordinary skill in the art.

It is generally desirable to provide a blunt distal tip 248 in an extension member, such as extension member 106'. RF tipping, or other conventional manufacturing techniques, is workable to form a desired blunt tip 248.

Actuation of the syringe 102 of FIG. 1 drives the fluid contained therein through the member 106 and through the atomizer portion 104.

Once being made aware of the instant disclosure, other and further ways of making, using, and assembling the apparatus will be readily apparent to those of ordinary skill in the art. Many of the components are readily commercially available or may be adapted from commercially available components.

## Claims

1. An apparatus (100) comprising:
an atomizing nozzle (104) component able to eject fluid substantially as a mist and affixed to an extension member (106) comprising a deformable element, wherein the extension member is effective to receive fluid communicating through the extension member from a fluid motive source; **characterised by**
a maximum characteristic size of a frontal area of the body (168) of the atomizing nozzle component is less than about 0.2 inch (0.5 cm), and
a ratio of the maximum characteristic size of a frontal area of the body (168) to a maximum characteristic size of a frontal area of the extension member is less than about 1.1, the apparatus further comprising a sleeve element structured to form a lap joint with respect to a distal end of the extension member (106).

2. The apparatus of claim 1, wherein:
the ratio of the maximum characteristic size of a frontal area of the body to the maximum characteristic size of the extension member is equal to about 1.0.

3. The apparatus of claim 1, wherein:
the ratio of the maximum characteristic size of a frontal area of the body to the maximum characteristic size of the extension member is less than 1.0.

4. The apparatus of claim 1, wherein:
the ratio of a maximum characteristic size of the extension member to a maximum characteristic size of a fluid ejection orifice of the atomizing nozzle component is less than 12.0.

5. The apparatus of claim 4, wherein:
an annular thickness of the sleeve element is less than about 0.008 inch (0.02 cm).

6. The apparatus of claim 4, wherein:
said sleeve element is structured to form a lap joint with respect to both the atomizing component and the extension member.

7. The apparatus of any one of claims 1 to 6, wherein:
a fluid transfer conduit inside the extension member is structured to form a lap joint with respect to an outside diameter of the atomizing component.

8. The apparatus of claim 1 or claim 7, wherein:
the deformable element being structured to permit a plastic deformation therein and to substantially hold a deformed shape in the extension member effective to orient a direction of a fluid discharge, from the atomizing component, relative to an axis of a proximal portion of the extension member.

9. The apparatus of any one of claims 1 to 8, further comprising:
a swirling chamber (196) disposed upstream of the orifice, the swirling chamber being configured to impart a transverse component of velocity to a distally discharged fluid stream, a proximal wall (200) of the swirling chamber comprising a fluid contact area having a wetted guide surface structured such that a vector normal to the surface gradually changes from pointing substantially in the transverse direction to pointing substantially in the distal direction as the vector progresses from a proximal position toward a distal position along the guide surface.

10. The apparatus of any one of claims 1 to 9, wherein:
the apparatus is disposable; and
the body has a maximum characteristic size of less than about 0.200 inch (0.5 cm).

11. The apparatus of claim 4, wherein: a ratio of the maximum characteristic size of a frontal area of the body to a maximum characteristic size of the ejection orifice is less than about 18.

## Patentansprüche

1. Vorrichtung (100), umfassend:
eine Zerstäubungsdüsenkomponente (104), die in der Lage ist, Fluid im Wesentlichen als Nebel auszustoßen und an einem Verlängerungselement (106) befestigt ist, das ein verformbares Element umfasst, wobei das Verlängerungselement wirksam ist, um Fluid aufzunehmen, das über das Verlängerungselement von einer Fluidantriebsquelle geleitet wird; **dadurch gekennzeichnet, dass**
eine maximale charakteristische Größe einer Stirnfläche des Körpers (168) der Zerstäubungsdüsenkomponente kleiner als etwa 0,2 Zoll (0,5 cm) ist, und
ein Verhältnis der maximalen charakteristischen Größe einer Stirnfläche des Körpers (168) zu einer maximalen charakteristischen Größe einer Stirnfläche des Verlängerungselements kleiner als etwa 1,1 ist, wobei die Vorrichtung ferner ein Hülsenelement umfasst, das strukturiert ist, um einen Überlappstoß in Bezug auf ein distales Ende des Verlängerungselements (106) zu bilden.

2. Vorrichtung nach Anspruch 1, wobei:
das Verhältnis der maximalen charakteristischen Größe einer Stirnfläche des Körpers zur maximalen charakteristischen Größe des Verlängerungselements gleich etwa 1,0 ist.

3. Vorrichtung nach Anspruch 1, wobei:
das Verhältnis der maximalen charakteristischen Größe einer Stirnfläche des Körpers zur maximalen charakteristischen Größe des Verlängerungselements kleiner als 1,0 ist.

4. Vorrichtung nach Anspruch 1, wobei:
das Verhältnis einer maximalen charakteristischen Größe des Verlängerungselements zu einer maximalen charakteristischen Größe einer Fluidausstoßöffnung der Zerstäubungsdüsenkomponente kleiner als 12,0 ist.

5. Vorrichtung nach Anspruch 4, wobei:
eine Ringdicke des Hülsenelements kleiner als etwa 0,008 Zoll (0,02 cm) ist.

6. Vorrichtung nach Anspruch 4, wobei:
das Hülsenelement strukturiert ist, um einen Überlappstoß sowohl in Bezug auf die Zerstäubungskomponente als auch auf das Verlängerungselement zu bilden,

7. Vorrichtung nach einem der Ansprüche 1 bis 6, wobei:
eine Fluidübertragungsleitung innerhalb des Verlängerungselements strukturiert ist, um einen Überlappstoß in Bezug auf einen Außendurchmesser der Zerstäubungskomponente zu bilden.

8. Vorrichtung nach Anspruch 1 oder Anspruch 7, wobei:
das verformbare Element strukturiert ist, um eine plastische Verformung darin zu ermöglichen und im Wesentlichen eine verformte Form in dem Verlängerungselement zu halten, die wirksam ist, um eine Richtung einer Fluidableitung aus der Zerstäubungskomponente in Bezug auf eine Achse eines proximalen Abschnitts des Verlängerungselements auszurichten.

9. Vorrichtung nach den Ansprüchen 1-8, ferner umfassend:
eine Wirbelkammer (196), die stromaufwärts der Öffnung angeordnet ist, wobei die Wirbelkammer konfiguriert ist, um einem distal abgeleiteten Fluidstrom eine transversale Geschwindigkeitskomponente zu verleihen, wobei eine proximale Wand (200) der Wirbelkammer eine Fluidkontaktfläche mit einer benetzten Führungsoberfläche umfasst, die so strukturiert ist, dass sich ein zur Oberfläche normaler Vektor allmählich von einem im Wesentlichen in Querrichtung weisenden Vektor zu einem im Wesentlichen in die distale Richtung weisenden Vektor ändert, wenn der Vektor von einer proximalen Position zu einer distalen Position entlang der Führungsoberfläche fortschreitet.

10. Vorrichtung nach einem der Ansprüche 1 bis 9, wobei:
die Vorrichtung entsorgbar ist; und
der Körper eine maximale charakteristische Größe von kleiner als etwa 0,200 Zoll (0,5 cm) aufweist.

11. Vorrichtung nach Anspruch 4, wobei:
ein Verhältnis der maximalen charakteristischen Größe einer Stirnfläche des Körpers zu einer maximalen charakteristischen Größe der Ausstoßöffnung kleiner als etwa 18 ist.

## Revendications

1. Appareil (100) comprenant :
un composant de buse d'atomisation (104) capable d'éjecter un fluide sensiblement sous forme de brouillard et fixé sur un élément d'extension (106) comprenant un élément déformable, l'élément d'extension étant efficace pour recevoir un fluide en communication à travers l'élément d'extension à partir d'une source motrice de fluide ; **caractérisé en ce que**
une taille caractéristique maximale d'une zone frontale du corps (168) du composant de buse d'atomisation est inférieure à environ 0,2 pouce (0,5 cm), et
un rapport de la taille caractéristique maximale d'une zone frontale du corps (168) à une taille caractéristique maximale d'une zone frontale de l'élément d'extension est inférieur à environ 1,1, l'appareil comprenant en outre un élément de manchon structuré pour former un joint à recouvrement par rapport à une extrémité distale de l'élément d'extension (106).

2. Appareil selon la revendication 1, dans lequel :
le rapport de la taille caractéristique maximale d'une zone frontale du corps à la taille caractéristique maximale de l'élément d'extension est égal à environ 1,0.

3. Appareil selon la revendication 1, dans lequel :
le rapport de la taille caractéristique maximale d'une zone frontale du corps à la taille caractéristique maximale de l'élément d'extension est inférieur à 1,0.

4. Appareil selon la revendication 1, dans lequel :
le rapport d'une taille caractéristique maximale de l'élément d'extension à une taille caractéristique maximale d'un orifice d'éjection de fluide du composant de buse d'atomisation est inférieur à 12,0.

5. Appareil selon la revendication 4, dans lequel :
une épaisseur annulaire de l'élément de manchon est inférieure à environ 0,008 pouce (0,02 cm).

6. Appareil selon la revendication 4, dans lequel :
ledit élément de manchon est structuré pour former un joint à recouvrement par rapport à la fois au composant d'atomisation et à l'élément d'extension.

7. Appareil selon l'une quelconque des revendications 1 à 6, dans lequel :
un conduit de transfert de fluide à l'intérieur de l'élément d'extension est structuré pour former un joint à recouvrement par rapport à un diamètre extérieur du composant d'atomisation.

8. Appareil selon la revendication 1 ou la revendication 7, dans lequel :
l'élément déformable est structuré pour permettre une déformation plastique dans celui-ci et pour sensiblement maintenir une forme déformée dans l'élément d'extension efficace pour orienter une direction d'une évacuation de fluide, à partir du composant d'atomisation, par rapport à un axe d'une partie proximale de l'élément d'extension.

9. Appareil selon l'une quelconque des revendications 1 à 8, comprenant en outre :
une chambre de tourbillonnement (196) disposée en amont de l'orifice, la chambre de tourbillonnement étant configurée pour conférer une composante transversale de vitesse à un courant de fluide évacué de manière distale, une paroi proximale (200) de la chambre de tourbillonnement comprenant une zone de contact de fluide ayant une surface de guidage humide structurée de telle sorte qu'un vecteur normal à la surface passe progressivement du fait de pointer sensiblement dans la direction transverse au fait de pointer sensiblement dans la direction distale à mesure que le vecteur progresse d'une position proximale vers une position distale le long de la surface de guidage.

10. Appareil selon l'une quelconque des revendications 1 à 9, dans lequel :
l'appareil est jetable ; et
le corps a une taille caractéristique maximale inférieure à environ 0,200 pouce (0,5 cm).

11. Appareil selon la revendication 4, dans lequel :
un rapport de la taille caractéristique maximale d'une zone frontale du corps à une taille caractéristique maximale de l'orifice d'éjection est inférieur à environ 18.
